# EUROPEAN PATENT SPECIFICATION

(11) **EP 3 515 554 B1**
(45) Date of publication and mention of the grant of the patent: **25.11.2026**
(21) Application number: 18851475.6
(22) Date of filing: 27.08.2018
(51) Int. Cl.: A61B 5/0507

(54) **APPARATUS OF MODULATING NEURONAL FIRING FREQUENCY AT BRAIN FUNCTIONAL SITE IN BRAIN**
VORRICHTUNG ZUR MODULATION DER NEURONALEN AUSLÖSUNGSFREQUENZ AN EINER GEHIRNFUNKTIONSSTELLE IM GEHIRN
APPAREIL DE MODULATION DE FRÉQUENCE NEURONALE D'ACTIVATION AU NIVEAU D'UN SITE FONCTIONNEL CÉRÉBRAL DANS LE CERVEAU

(30) Priority: 26.08.2017 US 201762550596 P; 29.10.2017 US 201762578463 P
(43) Date of publication of application: 31.07.2019
(73) Proprietor: Li, Xiaoping, Fukuoka-shi, Fukuoka 810-0021 (JP); Xia, Qian, Fukuoka-shi, Fukuoka 810-0021 (JP); Li, Jessica, Fukuoka-shi, Fukuoka 810-0021 (JP); Li, Joanna Le Xi, Fukuoka-shi, Fukuoka 810-0021 (JP); NewroCare Technology (Wuhan) Co., Ltd., Wuhan-city, Hubei Province, 430100 (CN)
(72) Inventor: Li, Xiaoping, Fukuoka-shi, Fukuoka 810-0021 (JP); Xia, Qian, Fukuoka-shi, Fukuoka 810-0021 (JP); Li, Jessica, Fukuoka-shi, Fukuoka 810-0021 (JP); Li, Joanna Le Xi, Fukuoka-shi, Fukuoka 810-0021 (JP)
(74) Representative: Brann AB
(86) International application number: PCT/CN2018/102586
(87) International publication number: WO 2019/042265

(56) References cited:
- WO-A1-2013/192582
- WO-A1-2017/076320
- WO-A2-2009/029141
- WO-A2-2010/149164
- US-A1- 2008 081 941
- US-A1- 2014 371 515
- US-A1- 2016 278 653
- US-A1- 2016 278 687

## Description

### Technical field

The present application relates to a method and an apparatus of modulating brain activities with electromagnetic field, particularly to a method and an apparatus of modulating a neuronal firing frequency at a brain functional site in a brain, in which an electromagnetic field is generated to modulate the neuronal firing frequency of brain functional sites to modulate the activities in the brain.

### Background

The brain is formed by neurons clustered into multiple brain functional sites. Each of the brain functional sites performs a particular function in the brain, and the function is characteristically related to the neuronal firing frequency at the brain functional site, showing as an activity in the brain. Thus, modulating the neuronal firing frequency at the brain functional site correspondingly modulate the activities in the brain. At each of the brain functional site, the neurons are surrounded by extracellular fluid that contains charged particles, such as calcium, potassium, and chloride. Each of the neurons has gates on its semipermeable membrane, through which the charged particles flow in or out of the neuron. As the charged particles flow (e.g. by diffusion) into and accumulate in the semipermeable membrane of the neuron and build up the membrane potential (i.e. polarization of the neuron) to a threshold value and the membrane receives a stimulation, the gates on the membrane open for all the charged particles to rush flow out of the membrane (causing depolarization of the neuron), forming a cycle of the polarization and depolarization of the neuron. At the end of the depolarization, another cycle of polarization of the neuron starts towards the next depolarization, ... and so on, forming the firing cycles of the neuron at a firing frequency determining the function state of the brain functional site.

The charged particles flowing by diffusion into the semipermeable membrane of the neuron to build up the membrane potential (i.e. polarization of the neuron) of the neuron depends on the concentration distribution of the charged particles in the surrounding extracellular fluid and therefore can be positively or negatively facilitated by varying the polarization density of the extracellular fluid. By varying the polarization density of the extracellular fluid, the diffusion rate can be varied and then the time taken for the polarization and depolarization of the neuron can be varied. Therefore, changing the variation frequency of the polarization density of the extracellular fluid changes the frequency of the polarization and depolarization cycle of the neuron, i.e. changes the firing rate of the neuron. WO2010149164A2 concerns an apparatus and a method for stimulating brain tissue with pulsed electromagnetic fields weaker than the limit for elicitation of the action potentials of the cells of the tissue to be stimulated. US20160278687A1 discloses methods of neural modulation using RF EM waves, in which the asymmetric pulse RF EM wave modulation of neuronal activation is selectively applied to a targeted brain functional site by having the frequency of the RF EM wave that is most sensitive to the targeted brain site, at which frequency of the RF EM wave the dielectric at the targeted brain site vary the EM wave propagating through it to the maximum.

### Summary

The invention is defined by the claims.

The near field of the electromagnetic field satisfies the condition of $0 < d \leq 0.62 ∗ \sqrt{\frac{{D}^{3}}{λ}}$, wherein d represents the distance from the antenna to an edge of the near field where the near field ends, D represents the largest dimension of the antenna, and λ represents the wavelength of the generated electromagnetic field in vacuum. Here, given that d satisfies the above condition, the electrical power from the antenna can be effectively transferred onto the targeted brain functional site within the near field through the inductive and capacitive effect of the current and charges in the antenna applied on the targeted brain functional site via the neuronal clusters formed by groups of neurons in the brain, which act as the secondary antennas for receiving the electrical power from the antenna to the targeted brain functional site within the near field.

The wavelength λ of the generated electromagnetic field in vacuum is such that the wavelength of the generated electromagnetic field at the brain functional site matches the dimension of the brain functional site. In this way, the brain functional site can be efficiently modulated by the electromagnetic field.

Additionally or alternatively, the wavelength λ of the generated electromagnetic field in vacuum may be such that the wavelength of the electromagnetic field at the brain functional site is from 70% to 130% of the dimension of the brain functional site.

The preset modulating frequency is determined based on the operational (or normal) neuronal firing frequency at the brain functional site and is preferably in the range of 1Hz to 2500Hz, preferably 5Hz to 125Hz, more preferably 5Hz to 45Hz, more preferably 25Hz to 85Hz, and more preferably 10Hz to 30Hz. When the preset modulating frequency is substantially the same as or approximate to the operational neuronal firing frequency at the brain functional site, the brain functional site can be modulated to operate properly at the operational neuronal firing frequency, to treat or prevent diseases at the brain functional site as well as to enhance brain functions.

The method may be used for treatment and prevention of one or more of insomnia, Alzheimer's disease, epilepsy, and depression, or for facilitation of the brain in learning and memorizing. In addition, the method may be further used for treatment and prevention of other diseases of the brain, by properly adjusting the wavelength λ of the generated electromagnetic field based on the dimension of the brain functional site and properly designating the preset modulating frequency based on the operational neuronal firing frequency at the brain functional site.

In an instance, when the brain functional site is a brain sleep prompting site, the method may further comprise:
- transforming the brain from its wake mode to its sleep mode by modulating the brain sleep promoting site at the preset modulating frequency with the power provided by the near field to have the brain itself remove the waste generated by the neurons in the wake mode of the brain, through the brain's glymphatic system that is working effectively only in the brain's sleep mode;
- further transforming the brain to a rapid eye movement (REM) sleep stage by modulating the brain sleep promoting site at the preset modulating frequency with the power provided by the near field; and
- once the brain has reached the REM sleep stage in its sleep mode, modulating one or more other brain functional sites, especially the functional sites involved in learning and memory, with another modulating frequency different from the preset modulating frequency to facilitate the brain's natural process of having the neurons involved in consumption of proteins complete their consumption through about the same neuronal connecting networking environment as they were working during the wake mode in consuming the proteins.

In another aspect, there is provided an apparatus of modulating a neuronal firing frequency at a brain functional site in a brain, comprising a signal generation module and an antenna connected to the signal generation module, wherein
the signal generation module is configured to generate an electrical signal with its power modulated with a preset modulating frequency in a burst format, and send the electrical signal to the antenna; and
the antenna is configured to receive the modulated electrical signal from the signal generation module and generate an electromagnetic field with its power in variation at the preset modulating frequency based on the electrical signal;
wherein when the apparatus is placed near the brain so that the targeted brain functional site is within the range of the near field of the electromagnetic field, extracellular fluid at the brain functional site is polarized with the power of the electromagnetic field, such that a polarization density of the extracellular fluid varies at the preset modulating frequency and neurons in the extracellular fluid are modulated to fire at the preset modulating frequency.

The near field of the electromagnetic field satisfies the condition of $0 < d \leq 0.62 ∗ \sqrt{\frac{{D}^{3}}{λ}}$, wherein d represents the distance from the antenna to an edge of the near field where the near field ends, D represents the largest dimension of the antenna, and λ represents the wavelength of the generated electromagnetic field in vacuum.

The wavelength λ of the generated electromagnetic field in vacuum is such that the wavelength of the electromagnetic field at the brain functional site matches the dimension of the brain functional site.

Additionally or alternatively, the wavelength λ of the generated electromagnetic field in vacuum is such that the wavelength of the electromagnetic field at the brain functional site is from 70% to 130% of the dimension of the brain functional site.

The preset modulating frequency is determined based on the operational (or normal) neuronal firing frequency at the brain functional site and is preferably in the range of 1Hz to 2500Hz, preferably 5Hz to 125Hz, more preferably 5Hz to 45Hz, more preferably 25Hz to 85Hz, and more preferably 10Hz to 30Hz.

In an instance, when the targeted brain functional site is a brain sleep prompting site, the apparatus may be used for:
- transforming the brain from its wake mode to its sleep mode by modulating the brain sleep promoting site at the preset modulating frequency with the power provided by the near field to have the brain itself remove the waste generated by the neurons in the wake mode of the brain, through the brain's glymphatic system that is working effectively only in the brain's sleep mode;
- further transforming the brain to a rapid eye movement (REM) sleep stage by modulating the brain sleep promoting site at a particular modulating frequency with the power provided by the near field; and
- once the brain has reached the REM sleep stage in its sleep mode, modulating one or more other brain functional sites, especially the functional sites involved in learning and memory, with another particular modulating frequency to facilitate the brain's natural process of having the neurons involved in consumption of proteins complete their consumption through about the same neuronal connecting networking environment as they were working during the wake mode in consuming the proteins.

As would be appreciated by those skilled in the art, the advantages achieved by the above method can also be achieved by the above apparatus.

### BRIEF DESCRIPTION OF DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

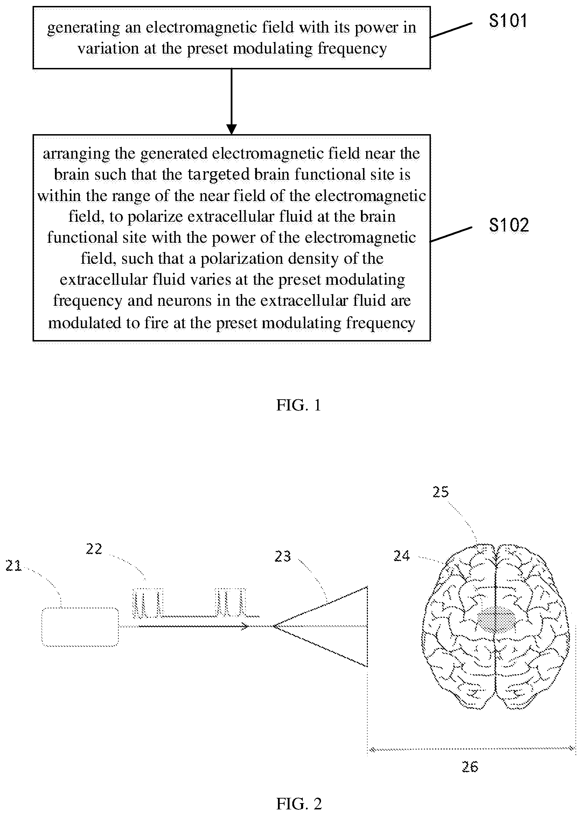

FIG. 1 is a schematic diagram showing a method of modulating a neuronal firing frequency at a brain functional site in a brain according to an embodiment of the present invention.
FIG. 2 schematically shows an apparatus of modulating a neuronal firing frequency at a brain functional site in a brain, in which a signal generation module generates and sends an electrical signal, e.g. a current signal with its amplitude modulated at a low frequency, to an antenna to generate an electromagnetic field with its near field acting on the brain to transform the brain from its wake mode to its sleep mode, according to an embodiment of the present invention.

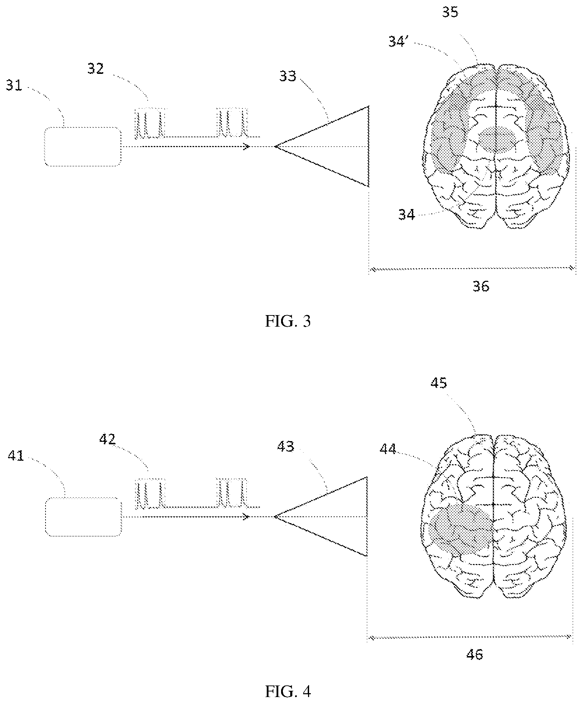

FIG. 3 schematically shows an apparatus of modulating a neuronal firing frequency at a brain functional site in a brain, in which a signal generation module generates and sends a series of electrical signals, e.g. current signals with its amplitude modulated at low frequencies, to an antenna to generate an electromagnetic field with its near field acting on the brain to facilitate the brain in its process of cleaning accumulated waste in the brain as well as completing the consumption of left over proteins surrounding the neurons for purposes of treatment and prevention of Alzheimer's disease, according to another embodiment of the present invention.
FIG. 4 schematically shows an apparatus of modulating a neuronal firing frequency at a brain functional site in a brain, in which a signal generation module generates and sends an electrical signal, e.g. a current signal with its amplitude modulated at a low frequency, to an antenna to generate an electromagnetic field with its near field acting on the brain to modulate the brain for treatment of epilepsy, according to another embodiment of the present invention.

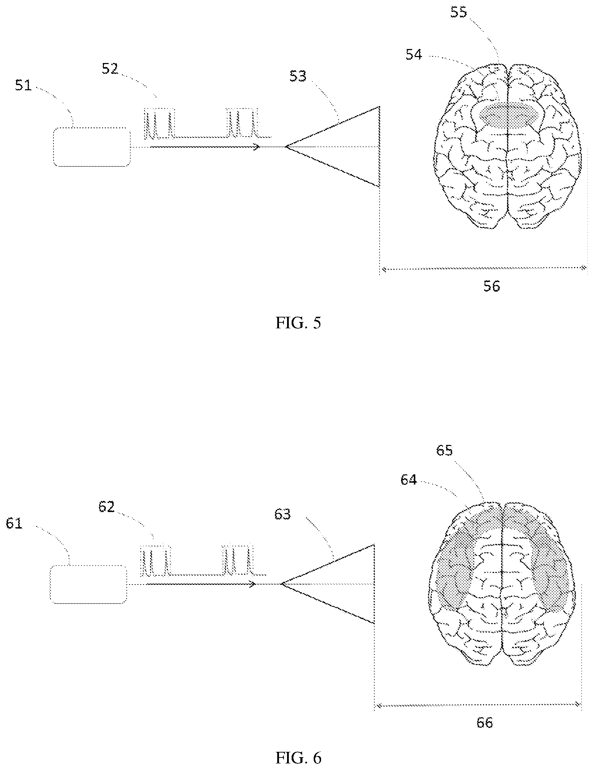

FIG. 5 schematically shows an apparatus of modulating a neuronal firing frequency at a brain functional site in a brain, in which a signal generation module generates and sends an electrical signal, e.g. a current signal with its amplitude modulated at a low frequency, to an antenna to generate an electromagnetic field with its near field acting on the brain functional sites that are responsible for serotonin release in the brain, including the SCG, to modulate the brain for treatment of depression, according to another embodiment of the present invention.
FIG. 6 schematically shows an apparatus of modulating a neuronal firing frequency at a brain functional site in a brain, in which a signal generation module generates and sends an electrical signal, e.g. a current signal with its amplitude modulated at a low frequency, to an antenna to generate an electromagnetic field with its near field acting on the brain functional sites that are responsible for learning and memorizing, including the frontal lobe and temporal lobe, to facilitate the brain in learning and memorizing, according to another embodiment of the present invention.

### DEAILED DESCRIPTION OF THE INVENTION

Some embodiments of the present invention will be described hereinafter with reference to the accompanying drawings.

In an embodiment, as shown in FIG. 1, a method of modulating a neuronal firing frequency at a brain functional site in a brain includes:
Step S101 of generating an electromagnetic field with its power in variation at the preset modulating frequency; and
Step S102 of arranging the generated electromagnetic field near the brain such that the targeted brain functional site is within the range of the near field of the electromagnetic field, to polarize extracellular fluid at the brain functional site with the power of the electromagnetic field, such that a polarization density of the extracellular fluid varies at the preset modulating frequency and neurons in the extracellular fluid are modulated to fire at the preset modulating frequency.

The near field of the electromagnetic field may be generated by an antenna and satisfy the condition of $0 < d \leq 0.62 ∗ \sqrt{\frac{{D}^{3}}{λ}}$, wherein d represents the distance from the antenna to an edge of the near field where the near field ends, D represents the largest dimension of the antenna, and *λ* represents the wavelength of the generated electromagnetic field in vacuum. Here, given that d satisfies the above condition, the electrical power from the antenna can be effectively transferred onto the targeted brain functional site within the near field through the inductive and capacitive effect of the current and charges in the antenna applied on the targeted brain functional site via the neuronal clusters formed by groups of neurons in the brain, which act as the secondary antennas for receiving the electrical power from the antenna to the targeted brain functional site within the near field.

As the brain is surrounded by the near field of the electromagnetic field generated by the antenna, through the strong inductive and capacitive effect of the currents and charges in the antenna, the neuronal clusters formed by groups of neurons in the brain act as the secondary antennas to receive the power from the antenna deep into the brain regions within the near field.

Here the near field is limited by the distance d from the antenna, the largest dimension D of the antenna, as well as the wavelength λ of the electromagnetic field generated by the antenna.

The wavelength λ of the generated electromagnetic field in vacuum is such that the wavelength of the generated electromagnetic field at the brain functional site matches the dimension of the brain functional site. In this way, the brain functional site can be efficiently modulated by the electromagnetic field.

Additionally or alternatively, the wavelength λ of the generated electromagnetic field in vacuum may be such that the wavelength of the electromagnetic field at the brain functional site is from 70% to 130% of the dimension of the brain functional site.

For example, in the case of selectively modulating the sleep promoting site ventrolateral preoptic nucleus (VLPO) in the brain, given that the dimension of the VLPO is about 1 cm and an electromagnetic field of 6 cm wavelength in vacuum will have a wavelength of 1 cm in the brain, the 1 cm wavelength matches the dimension of the VLPO for high modulation efficiency. In this case, the wavelength λ of the electromagnetic field is 6 cm and thus the frequency of the electromagnetic field is 5GHz, and the largest dimension D of the antenna is 18 cm, then $0 < d \leq 0.62 ∗ \sqrt{\frac{{18}^{3}}{6}} = 19.3 cm$

That is, the maximum distance of the near field is 19.3 cm from the antenna. Here, the value of the dimension D relates to the value of the distance d, and those skilled in the art knows proper adjustment of the values of D and d correlatively to properly acting the targeted brain functional site.

In the present embodiment, the electromagnetic field is generated through the antenna, where the antenna is placed near the brain such that the targeted functional site of the brain is within the near field of the generated electromagnetic field and the extracellular fluid in the object functional site is polarized by the electric field of the near field, and the power of the near field is modulated to appear in bursts with the preset modulating frequency such that the polarization density of the extracellular fluid in the object functional site varies at the same frequency as the variation frequency of the power (i.e. the preset modulating frequency), thereby modulating the neuronal firing frequency at the targeted brain functional site.

In an example of the above method, the brain is transformed from its wake mode to its sleep mode, in which an antenna generates an electromagnetic field with its near field acting on the brain for sleep induction. During operation, the antenna is located near the brain and the sleep promoting sites of the brain, including the ventrolateral preoptic nucleus (VLPO), is within the near field of the generated electromagnetic field and the extracellular fluid in the object functional site is polarized by the power of the near field, where the power of the electromagnetic field is modulated with a low frequency in a burst format such that the polarization density of the extracellular fluid in the object functional site varies at the same frequency as the variation frequency of the power, thereby modulating the neuronal firing frequency at the brain sleep promoting site (including modulating the VLPO to a certain frequency), inducing the brain to transform from the wake mode to the sleep mode. Herein, the frequency of the generated electromagnetic field is determined such that the wavelength of the generated electromagnetic field in the sleep-promoting site in the brain best matches the dimensions of the sleep-promoting site so that the modulating effect of the generated electromagnetic field is selective to the sleep-promoting site. The frequency of the generated electromagnetic field is in the range of 1GHz to 20GHz, preferably in the range of 4GHz to 6GHz. The modulating frequency for the variation of the power of the generated electromagnetic field is in the range of 5Hz to 2500Hz, preferably in the range of 10Hz to 30Hz.

In another example of the above method for the treatment and prevention of Alzheimer's disease, an antenna generates an electromagnetic field with its near field acting on the brain, to implement two sequential steps of: 1) transforming the brain from its wake mode to its sleep mode to have the brain itself remove the waste generated by the neurons in the wake mode of the brain, through the brain's glymphatic system that is working effectively only in the brain's sleep mode; 2) transforming the brain from its wake mode to its sleep mode up the rapid eye movement (REM) sleep stage by modulating the brain sleep promoting site at the preset modulating frequency with the power provided by the near field; and 3) once the brain has reached the REM sleep stage in its sleep mode, modulating one or more other brain functional sites, especially the functional sites involved in learning and memory, with another particular modulating frequency different from the preset modulating frequency to facilitate the brain's natural process of having the neurons involved in consumption of certain proteins, such as amyloid beta, complete their consumption through substantially the same neuronal connecting networking environment as they were working during the brain wake mode in consuming the proteins. During the operation, the antenna is located near the brain and all the functional sites of the brain, especially the brain functional sites for sleep promoting and for learning and memorizing, are within the near field of the generated electromagnetic field and the extracellular fluid in all the brain functional sites is polarized by the near field, and the power of the near field is modulated with the low preset modulating frequency in a burst format such that the polarization densities of the extracellular fluid in the brain functional sites vary at the same frequency as the variation frequency of the power of the electromagnetic field, thereby modulating the neuronal firing frequency at the brain functional sites to the preset modulating frequency to transform the brain from wake mode to sleep mode, and then modulating the neuronal firing frequency to another frequency to facilitate the brain in its natural process of dealing with the left over proteins during the brain in wakefulness, which are the causes of Alzheimer's disease. The frequency of the generated electromagnetic field is determined such that the wavelength of the generated electromagnetic field in the sleep-promoting site best matches the dimensions of the sleep-promoting site as well as the learning and memorizing sites in the brain so that the modulating effect of the generated electromagnetic field is selective to the sleep-promoting site as well as the learning and memorizing sites. The frequency of the electromagnetic field is in the range of 1GHz to 20GHz, preferably in the range of 4GHz to 6GHz. The modulating frequency for the variation of the power of the generated electromagnetic field is in the range of 5Hz to 2500Hz, preferably in the range of 10Hz to 30Hz.

In another example of the above method, an antenna generates an electromagnetic field having power varying at the preset modulating frequency, with its near field acting on the brain. During the operation, the antenna is located near the brain and the part of the brain that is epileptically diseased is within the near field of the generated electromagnetic field and the extracellular fluid in this part of the brain is polarized by the near field; the power of the near field is modulated with a low frequency (i.e. the preset modulating frequency) in a burst format to make the polarization densities of the extracellular fluid in the brain functional sites vary at the same frequency as the variation frequency of the power, thereby modulating the neurons at the epileptically diseased part of the brain to fire at the normal frequency when this part of the brain is not epileptically diseased, until the epileptically diseased neurons have been rehabilitated to normal in their firing and the epilepsy is cured. The frequency of the generated electromagnetic field is determined such that the wavelength of the generated electromagnetic field at the epileptically diseased part of the brain best matches the dimensions of the epileptically diseased part of the brain so that the modulating effect of the generated electromagnetic field is selective to the epileptically diseased part of the brain. The frequency of the electromagnetic field is in the range of 300MHz to 20GHz. The modulating frequency for the variation of the power of the generated electromagnetic field is in the range of 1Hz to 2500Hz with the optimum in the range of 5Hz to 45Hz.

In another example of the above method to modulate the brain for treatment of depression, a current signal with its amplitude modulated at a low frequency is transferred to an antenna to generate an electromagnetic field with its near field acting on the brain functional sites that are responsible for serotonin (i.e. diminished activity of serotonin pathways plays a causal role in the pathophysiology of depression) release in the brain, including the subcallosal cingulate gyrus (SCG). During the operation, the antenna is located near the brain and the part of the brain that is responsible for serotonin release is within the near field of the generated electromagnetic field and the extracellular fluid in this part of the brain is polarized by the near field; the power of the near field is modulated with a low frequency in a burst format to make the polarization densities of the extracellular fluid in the brain functional sites vary at the same frequency as the variation frequency of the power, thereby modulating the neuronal firing frequency at the part of the brain to fire at the frequency they normally do when the brain is free of depression. In this way the neurons in the part of the brain are rehabilitated back to their normal firing behaviors and the depression in the brain is cured. Here, the frequency of the generated electromagnetic field is determined such that the wavelength of the generated electromagnetic field in the brain functional sites that are responsible for serotonin release in the brain best matches the dimensions of the brain functional sites, including the subcallosal cingulate gyrus (SCG) so that the modulating effect of the generated electromagnetic field is selective to these brain functional sites. The frequency of the electromagnetic frequency may be in the range of 1GHz to 20GHz. The modulating frequency for the variation of the power of the generated electromagnetic field is in the range of 1Hz to 2500Hz, preferably in the range of 5Hz to 45Hz.

In another example of the above method which is used to facilitate the brain in learning and memorizing, a current signal with its amplitude modulated at a low frequency is transferred to an antenna to generate an electromagnetic field with its near field acting on the brain functional sites that are responsible for learning and memorizing, including the frontal lobe and temporal lobe. During the operation, the antenna is placed near the concerned brain functional sites such that the brain functional sites are within the near field of the generated electromagnetic field and the extracellular fluid in this part of the brain is polarized by the near field; the power of the near field is modulated with a low frequency in a burst format to make the polarization densities of the extracellular fluid in the brain functional sites vary at the same frequency as the variation frequency of the power, thereby modulating all the neurons in the concerned brain functional sites to fire at a critical frequency for binding between the neurons via their synapses. With the modulation much more synapses of the neurons in the concerned brain functional sites are activated/alerted for binding such that the brain learns faster and memorizing more solidly. In the example, the frequency of the generated electromagnetic field is determined such that the wavelength of the generated electromagnetic field at the brain functional sites that are responsible for learning and memorizing best matches the dimensions of the brain functional sites so that the modulating effect of the generated electromagnetic field is selective to these brain functional sites. The frequency of the electromagnetic field is in the range of 300MHz to 20GHz, preferably in the range of 2GHz to 10GHz. The modulating frequency for the variation of the power of the generated electromagnetic field is in the range of 1Hz to 2500Hz, preferably in the range of 25Hz to 85Hz.

Likewise, the above method can be used for the treatment and prevention of insomnia.

As can be seen, the frequency (or the wavelength λ) of the generated electromagnetic field in vacuum is determined depending on the dimension of the targeted brain functional site, and the preset modulating frequency for modulating the power of the electromagnetic field is determined based on the operational neuronal firing frequency at the brain functional site and may be in the range of 1Hz to 2500Hz, preferably 5Hz to 125Hz, more preferably 5Hz to 45Hz, more preferably 25Hz to 85Hz, and more preferably 10Hz to 30Hz.

The method can be used for treatment and prevention of one or more of insomnia, Alzheimer's disease, epilepsy, and depression, or for facilitation of the brain in learning and memorizing.

In another embodiment, there is provided an apparatus of modulating a neuronal firing frequency at a brain functional site in a brain. Various examples of the apparatus are illustratively described below.

As shown in FIG. 2, an apparatus of the present embodiment, which is for example used for sleep induction, includes a signal generation module 21 and an antenna 23 connected to the signal generation module 21. The signal generation module 21 is configured to generate a current signal with its amplitude modulated with a preset low modulating frequency in a burst format 22 and send the current signal to the antenna 23. Therefore, the antenna 23 receives the modulated electrical signal from the signal generation module 21 and generates an electromagnetic field with its near field range 26 covering the sleep promoting site 24 of the brain 25, polarizing the extracellular fluid in the sleep promoting site 24 and inducing a variation of the polarization density at the modulating frequency such that the neuronal firing frequency is modulated to the modulated frequency for transforming the brain 25 from wake to sleep. Here, the electromagnetic field has power varying at the preset modulating frequency.

As shown in FIG. 3, an apparatus of the present embodiment, which is used for treatment and prevention of Alzheimer's disease, includes a signal generation module 31 and an antenna 33 connected to the signal generation module 31. The signal generation module 31 is configured to generate a current signal with its amplitude modulated with a preset low modulating frequency in a burst format 32 and send the current signal to the antenna 33. The antenna 33 receives the modulated electrical signal from the signal generation module 31 and generates an electromagnetic field with its near field range 36 covering the sleep promoting site 34 and the learning and memorizing sites 34' as well as other sites of the brain 35, thereby firstly polarizing the extracellular fluid in the brain functional sites and inducing variation of the polarization density at the brain functional sites with the preset modulating frequency such that the neuronal firing at the sleep promoting site 34 is modulated to transform the brain 35 from wake to sleep to facilitate the glymphatic system removal of waste generated by the neurons in the brain 35; and then, as the brain 35 is in its sleep mode, inducing the polarization density at the learning and memorizing sites 34' as well as other sites of the brain 35 to vary at another particular frequency such that the neuronal firing at the learning and memorizing sites 34' as well as other sites of the brain 35 is modulated to the another particular frequency that facilitates synaptic connections between the neurons in consumption/absorption of the leftover proteins from the wake mode of the brain 35; both of these facilitation processes provide the effect of treatment and prevention of Alzheimer's disease to the brain 35.

As shown in FIG. 4, an apparatus of the present embodiment, which is used for treatment of epilepsy, includes a signal generation module 41 and an antenna 43 connected to the signal generation module 41. The signal generation module 41 is configured to generate a current signal with its amplitude modulated with a preset low modulating frequency in a burst format 42 and send the current signal to the antenna 43. The antenna 43 receives the modulated electrical signal from the signal generation module 41 and generates an electromagnetic field with its near field range 46 covering the epileptically diseased part 44 of the brain 45, thereby polarizing the extracellular fluid in the epileptically diseased part 44 of the brain 45 and inducing a variation of the polarization density at the modulating frequency that is the normal neuronal firing frequency for this part 44 of the brain 45 when it is not epileptically diseased to modulate the neurons in the epileptically diseased part 44 to fire at the modulating frequency such that the neurons are rehabilitated to normal in their neuronal firing behavior and the epilepsy in the brain 45 is cured.

As shown in FIG. 5, an apparatus of the present embodiment, which is used for treatment of depression, includes a signal generation module 51 and an antenna 53 connected to the signal generation module 51. The signal generation module 51 is configured to generate a current signal with its amplitude modulated with a preset low modulating frequency in a burst format 52 and send the current signal to the antenna 53. The antenna 53 receives the modulated electrical signal from the signal generation module 51 and generates an electromagnetic field with its near field range 56 covering the brain functional sites that are responsible for serotonin release in the brain 55, including the SCG 54, thereby polarizing the extracellular fluid in the SCG 54 as well as the brain functional sites that are responsible for serotonin release in the brain 55, and inducing a variation of the polarization density at the modulating frequency that is the normal firing frequency of these brain functional sites when the brain 55 is free of depression, rehabilitating the neurons in these brain functional sites to their normal firing behaviors such that the depression in the brain 55 is cured.

As shown in FIG. 6, an apparatus of the present embodiment, which is used for treatment of depression, includes a signal generation module 61 and an antenna 63 connected to the signal generation module 61. The signal generation module 61 is configured to generate a current signal with its amplitude modulated with a preset low modulating frequency in a burst format 62 and send the current signal to the antenna 63. The antenna 63 receives the modulated electrical signal from the signal generation module 61 and generates an electromagnetic field with its near field range 66 covering the learning and memorizing sites 64 as well as other brain functional sites in the brain 65, polarizing the extracellular fluid in the learning and memorizing sites 64 as well as other brain functional sites in the brain 65, and inducing a variation of the polarization density at a modulating frequency to modulate all the neurons in the concerned brain functional sites fire at a critical frequency for binding between the neurons via their synapses, activating much more than usual the synapses of the neurons in the concerned brain functional sites for binding such that the brain 65 learns faster and memorizing more solidly.

As to the apparatus in the above examples, the near field of the electromagnetic field satisfies the condition of $0 < d \leq 0.62 ∗ \sqrt{\frac{{D}^{3}}{λ}}$, wherein d represents the distance from the antenna to an edge of the near field where the near field ends, D represents the largest dimension of the antenna, and λ represents the wavelength of the generated electromagnetic field in vacuum.

The wavelength λ of the generated electromagnetic field in vacuum is such that the wavelength of the electromagnetic field at the brain functional site matches the dimension of the brain functional site.

Additionally or alternatively, the wavelength λ of the generated electromagnetic field in vacuum is such that the wavelength of the electromagnetic field at the brain functional site is from 70% to 130% of the dimension of the brain functional site.

The preset modulating frequency is determined based on the operational (or normal) neuronal firing frequency at the brain functional site and is preferably in the range of 1Hz to 2500Hz, preferably 5Hz to 125Hz, more preferably 5Hz to 45Hz, more preferably 25Hz to 85Hz, and more preferably 10Hz to 30Hz.

In the above embodiments of the present invention, the waveform of the electromagnetic field generated by the antenna may be of any waveform, including but not limited to alternating waveform and pulse waveform.

While the invention is susceptible to various modifications and alternative forms, specific embodiments thereof have been shown by way of example in the drawings and the above detailed description. It should be understood, however, that it is not intended to limit the invention to the particular forms disclosed, but on the contrary, the intention is to cover all modifications, and alternatives falling within the scope of the invention as defined by the appended claims.

It is expected that during the life of a patent maturing from this application many relevant disinfecting connectors will be developed; the scope of the term disinfecting connector is intended to include all such new technologies *a priori.*

The terms "comprises", "comprising", "includes", "including", "has", "having" and their conjugates mean "including but not limited to".

As used herein, the singular forms "a", "an" and "the" include plural references unless the context clearly dictates otherwise.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

## Claims

1. An apparatus for modulation of neuronal firing at a brain functional site (24, 34, 34', 44, 54, 64) in a brain (25, 35, 45, 55, 65), comprising a signal generation module (21, 31, 41, 51, 61) and an antenna (23, 33, 43, 53, 63) connected to the signal generation module (21, 31, 41, 51, 61), wherein
the signal generation module (21, 31, 41, 51, 61) is configured to generate an electrical signal with its power modulated with a preset modulating frequency in a burst format (22, 32, 42, 52, 62), and send the electrical signal to the antenna (23, 33, 43, 53, 63); and
the antenna (23, 33, 43, 53, 63) is configured to receive the modulated electrical signal from the signal generation module (21, 31, 41, 51, 61) and generate an electromagnetic field with its power in variation at the preset modulating frequency based on the electrical signal, to polarize extracellular fluid at the brain functional site (24, 34, 34', 44, 54, 64) placed within the range (26) of a near field of the electromagnetic field with the power of the electromagnetic field, for causing a polarization density of the extracellular fluid to vary at the preset modulating frequency and thus causing neurons in the extracellular fluid to be modulated to fire at the preset modulating frequency,
wherein the preset modulating frequency is determined based on a normal neuronal firing frequency of the brain functional site (24, 34, 34', 44, 54, 64), and the near field of the electromagnetic field satisfies the condition of $0 < d \leq 0.62 ∗ \sqrt{\frac{{D}^{3}}{λ}}$, wherein d represents the distance from the antenna (23, 33, 43, 53, 63) to an edge of the near field where the near field ends, D represents the largest dimension of the antenna (23, 33, 43, 53, 63), and λ represents the wavelength of the generated electromagnetic field in vacuum.

2. The apparatus of claim 1, wherein the wavelength λ of the generated electromagnetic field in vacuum is such that the wavelength of the electromagnetic field at the brain functional site (24, 34, 34', 44, 54, 64) matches the dimension of the brain functional site (24, 34, 34', 44, 54, 64).

3. The apparatus of claim 2, wherein the wavelength λ of the generated electromagnetic field in vacuum is such that the wavelength of the electromagnetic field at the brain functional site (24, 34, 34', 44, 54, 64) is from 70% to 130% of the dimension of the brain functional site (24, 34, 34', 44, 54, 64).

4. The apparatus of claim 1, wherein the preset modulating frequency is in the range of 1Hz to 2500Hz, preferably 5Hz to 125Hz, more preferably 5Hz to 45Hz, more preferably 25Hz to 85Hz, and more preferably 10Hz to 30Hz.

5. The apparatus of claim 1, wherein the brain functional site is a brain sleep promoting site (34), and the apparatus is adapted to be used for:
- transforming the brain (35) from its wake mode to its sleep mode by modulating the brain sleep promoting site (34) at a first preset modulating frequency determined based on the operational neuronal firing frequency of the brain sleep prompting site with the power provided by the near field to have the brain (35) itself remove the waste generated by the neurons in the wake mode of the brain (35), through the brain's glymphatic system that is working effectively only in the brain's sleep mode;
- further transforming the brain (35) to a rapid eye movement (REM) sleep stage by modulating the brain sleep promoting site (34) at the first preset modulating frequency with the power provided by the near field; and
once the brain (35) has reached the REM sleep stage in its sleep mode, modulating other brain functional sites, especially the functional sites (34') involved in learning and memorizing, with a second preset modulating frequency that is different from the preset modulating frequency and determined based on the operational neuronal firing frequency of the one or more other brain functional sites to facilitate the brain's natural process of having the neurons involved in consumption of proteins complete their consumption through about the same neuronal connecting networking environment as they were working during the wake mode in consuming the proteins.

## Patentansprüche

1. Vorrichtung zur Modulation der neuronalen Aktivität an einer Gehirnfunktionsstelle (24, 34, 34', 44, 54, 64) in einem Gehirn (25, 35, 45, 55, 65), umfassend ein Signalerzeugungsmodul (21, 31, 41, 51, 61) und eine mit dem Signalerzeugungsmodul (21, 31, 41, 51, 61) verbundene Antenne (23, 33, 43, 53, 63), wobei
das Signalerzeugungsmodul (21, 31, 41, 51, 61) dazu konfiguriert ist, ein elektrisches Signal zu erzeugen, dessen Leistung mit einer voreingestellten Modulationsfrequenz in einem Burst-Format (22, 32, 42, 52, 62) moduliert ist, und das elektrische Signal an die Antenne (23, 33, 43, 53, 63) zu senden; und
die Antenne (23, 33, 43, 53, 63) dazu konfiguriert ist, das modulierte elektrische Signal von dem Signalerzeugungsmodul (21, 31, 41, 51, 61) zu empfangen und basierend auf dem elektrischen Signal ein elektromagnetisches Feld zu erzeugen, dessen Leistung bei der voreingestellten Modulationsfrequenz variiert, um extrazelluläres Fluid an der Gehirnfunktionsstelle (24, 34, 34', 44, 54, 64), die sich innerhalb des Bereichs (26) eines Nahfeldes des elektromagnetischen Feldes befindet, mit der Leistung des elektromagnetischen Feldes zu polarisieren, um zu bewirken, dass Polarisationsdichte des extrazellulären Fluids bei der voreingestellten Modulationsfrequenz variiert, und so zu bewirken, dass Neuronen in dem extrazellulären Fluid moduliert werden, um bei der voreingestellten Modulationsfrequenz aktiviert zu werden,
wobei die voreingestellte Modulationsfrequenz basierend auf einer normalen neuronalen Auslösungsfrequenz der Gehirnfunktionsstelle (24, 34, 34', 44, 54, 64) bestimmt wird und das Nahfeld des elektromagnetischen Feldes die Bedingung $0 < d \leq 0 , 62 ∗ \sqrt{\frac{{D}^{3}}{λ}}$ erfüllt, wobei d den Abstand von der Antenne (23, 33, 43, 53, 63) zu einer Kante des Nahfeldes darstellt, an der das Nahfeld endet, D die größte Abmessung der Antenne (23, 33, 43, 53, 63) darstellt und λ die Wellenlänge des erzeugten elektromagnetischen Feldes im Vakuum darstellt.

2. Vorrichtung nach Anspruch 1, wobei die Wellenlänge λ des erzeugten elektromagnetischen Feldes im Vakuum derart ist, dass die Wellenlänge des elektromagnetischen Feldes an der Gehirnfunktionsstelle (24, 34, 34', 44, 54, 64) der Abmessung der Gehirnfunktionsstelle (24, 34, 34', 44, 54, 64) entspricht.

3. Vorrichtung nach Anspruch 2, wobei die Wellenlänge λ des erzeugten elektromagnetischen Feldes im Vakuum derart ist, dass die Wellenlänge des elektromagnetischen Feldes an der Gehirnfunktionsstelle (24, 34, 34', 44, 54, 64) 70 % bis 130 % der Abmessung der Gehirnfunktionsstelle (24, 34, 34', 44, 54, 64) beträgt.

4. Vorrichtung nach Anspruch 1, wobei die voreingestellte Modulationsfrequenz in dem Bereich von 1 Hz bis 2500 Hz, vorzugsweise 5 Hz bis 125 Hz, noch bevorzugter 5 Hz bis 45 Hz, noch bevorzugter 25 Hz bis 85 Hz und noch bevorzugter 10 Hz bis 30 Hz liegt.

5. Vorrichtung nach Anspruch 1, wobei es sich bei der Gehirnfunktionsstelle um eine schlaffördernden Gehirnstelle (34) handelt und die Vorrichtung dazu ausgelegt ist, für Folgendes verwendet zu werden:
- Überführen des Gehirns (35) von seinem Wachmodus in seinen Schlafmodus durch Modulieren der schlaffördernden Gehirnstelle (34) bei einer ersten voreingestellten Modulationsfrequenz, die basierend auf der funktionellen neuronalen Auslösungsfrequenz der schlafanregenden Gehirnstelle bestimmt wird, mit der von dem Nahfeld bereitgestellten Leistung, damit das Gehirn (35) selbst die von den Neuronen in dem Wachzustand des Gehirns (35) erzeugten Abfallstoffe über das glymphatische System des Gehirns entfernt, das nur im Schlafmodus des Gehirns effektiv arbeitet;
- ferner Überführen des Gehirns (35) in ein Schlafstadium der schnellen Augenbewegung (REM) durch Modulieren der schlaffördernde Gehirnstelle (34) bei der ersten voreingestellten Modulationsfrequenz mit der von dem Nahfeld bereitgestellten Leistung; und
sobald das Gehirn (35) in seinem Schlafmodus das REM-Schlafstadium erreicht hat, Modulieren anderer Gehirnfunktionsstellen, insbesondere der am Lernen und Erinnern beteiligten Funktionsstellen (34'), bei einer zweiten voreingestellten Modulationsfrequenz, die sich von der voreingestellten Modulationsfrequenz unterscheidet und basierend auf der funktionellen neuronalen Auslösungsfrequenz der einen oder der mehreren anderen Gehirnfunktionsstellen bestimmt wird, um den natürlichen Prozess des Gehirns zu erleichtern, bei dem die an dem Verbrauch von Proteinen beteiligten Neuronen ihren Verbrauch über etwa die gleiche neuronale verbindende Netzwerkumgebung abschließen, in der sie während des Wachmodus beim Verbrauchen der Proteine gearbeitet haben.

## Revendications

1. Appareil de modulation d'activation neuronale au niveau d'un site fonctionnel cérébral (24, 34, 34', 44, 54, 64) dans un cerveau (25, 35, 45, 55, 65), comprenant un module de génération de signal (21, 31, 41, 51, 61) et une antenne (23, 33, 43, 53, 63) connectée au module de génération de signal (21, 31, 41, 51, 61), dans lequel
le module de génération de signal (21, 31, 41, 51, 61) est configuré pour générer un signal électrique dont la puissance est modulée avec une fréquence de modulation prédéfinie sous forme de rafale (22, 32, 42, 52, 62), et pour envoyer le signal électrique à l'antenne (23, 33, 43, 53, 63) ; et
l'antenne (23, 33, 43, 53, 63) est configurée pour recevoir le signal électrique modulé provenant du module de génération de signal (21, 31, 41, 51, 61) et générer un champ électromagnétique dont la puissance varie à la fréquence de modulation prédéfinie en fonction du signal électrique, afin de polariser le fluide extracellulaire au niveau du site fonctionnel cérébral (24, 34, 34', 44, 54, 64) placé dans la zone (26) d'un champ proche du champ électromagnétique, avec la puissance du champ électromagnétique, pour entraîner une variation d'une densité de polarisation du fluide extracellulaire à la fréquence de modulation prédéfinie et ainsi entraîner la modulation de l'activation des neurones dans le fluide extracellulaire à la fréquence de modulation prédéfinie,
dans lequel la fréquence de modulation prédéfinie est déterminée en fonction d'une fréquence d'activation neuronale normale du site fonctionnel cérébral (24, 34, 34', 44, 54, 64), et le champ proche du champ électromagnétique satisfait la condition $0 < d \leq 0 , 62 ∗ \sqrt{\frac{{D}^{3}}{λ}}$ , où d représente la distance de l'antenne (23, 33, 43, 53, 63) à un bord du champ proche où le champ proche se termine, D représente la plus grande dimension de l'antenne (23, 33, 43, 53, 63), et λ représente la longueur d'onde du champ électromagnétique généré dans le vide.

2. Appareil selon la revendication 1, dans lequel la longueur d'onde λ du champ électromagnétique généré dans le vide est telle que la longueur d'onde du champ électromagnétique au niveau du site fonctionnel cérébral (24, 34, 34', 44, 54, 64) correspond à la dimension du site fonctionnel cérébral (24, 34, 34', 44, 54, 64).

3. Appareil selon la revendication 2, dans lequel la longueur d'onde λ du champ électromagnétique généré dans le vide est telle que la longueur d'onde du champ électromagnétique au niveau du site fonctionnel cérébral (24, 34, 34', 44, 54, 64) représente entre 70 % et 130 % de la dimension du site fonctionnel cérébral (24, 34, 34', 44, 54, 64).

4. Appareil selon la revendication 1, dans lequel la fréquence de modulation prédéfinie est comprise entre 1 Hz et 2 500 Hz, de préférence entre 5 Hz et 125 Hz, plus préférablement entre 5 Hz et 45 Hz, plus préférablement entre 25 Hz et 85 Hz, et plus préférablement entre 10 Hz et 30 Hz.

5. Appareil selon la revendication 1, dans lequel le site fonctionnel cérébral est un site favorisant le sommeil cérébral (34), et l'appareil est conçu pour être utilisé pour :
- transformer le cerveau (35) de son mode d'éveil à son mode de sommeil en modulant le site favorisant le sommeil cérébral (34) à une première fréquence de modulation prédéfinie déterminée en fonction de la fréquence d'activation neuronale opérationnelle du site d'incitation au sommeil cérébral avec la puissance fournie par le champ proche pour que le cerveau (35) élimine lui-même les déchets générés par les neurones dans le mode d'éveil du cerveau (35), par l'intermédiaire du système glymphatique du cerveau qui ne fonctionne efficacement que dans le mode de sommeil du cerveau ;
- transformer davantage le cerveau (35) en une phase de sommeil paradoxal (REM) en modulant le site favorisant le sommeil cérébral (34) à la première fréquence de modulation prédéfinie avec la puissance fournie par le champ proche ; et
une fois que le cerveau (35) a atteint la phase de sommeil paradoxal dans son mode de sommeil, la modulation d'autres sites fonctionnels cérébraux, en particulier les sites fonctionnels (34') impliqués dans l'apprentissage et la mémorisation, avec une seconde fréquence de modulation prédéfinie différente de la fréquence de modulation prédéfinie et déterminée en fonction de la fréquence d'activation neuronale opérationnelle d'un ou de plusieurs autres sites fonctionnels cérébraux pour faciliter le processus naturel du cerveau permettant aux neurones impliqués dans la consommation de protéines de terminer leur consommation à travers un environnement de réseau de connexion neuronale à peu près identique à celui dans lequel ils travaillaient pendant le mode d'éveil lors de la consommation des protéines.
